# EUROPEAN PATENT APPLICATION

(11) **EP 1 829 559 A1**
(43) Date of publication of application: **05.09.2007**
(21) Application number: 07250828.6
(22) Date of filing: 28.02.2007
(51) Int. Cl.: A61L 9/013, C11B 9/00, A61P 25/20

(54) **Method for improving sleep behaviors**

(30) Priority: 01.03.2006 US 366778
(71) Applicant: Johnson & Johnson Consumer Companies, Inc., Skillman, NJ 53008 (US)
(72) Inventor: Luedtke, Kathryn, Doylestown PA 18901 (US); Wiegand, Benjamin, Yardley PA, 19067 (US)
(74) Representative: James, Anthony Christopher W.P.

(57) **Abstract**

The invention relates to a method for improving the sleep behaviors of a human comprising the administration of a sensory regimen which comprises:
a. the administration of a first sensory experience comprising topically applying to the skin or hair of the human a cleansing product containing an effective amount of a sensory fragrance; and
b. after step (a) the administration of a second sensory experience comprising administering an effective amount of a sensory fragrance.

The sensory fragrance used in step (a) and the sensory fragrance used in step (b) can be the same or different and is capable of reducing alpha activity.

## Description

### 1. FIELD OF THE INVENTION

The invention relates to a method for improving sleep behaviors of a human comprising the administration of a sensory regimen which comprises the administration of a sensory fragrance.

### 2. BACKGROUND AND DESCRIPTION OF THE PRIOR ART

Due to many factors, e.g., stress in our daily lives, a large portion of the population exhibit symptoms related to sleep difficulties. Sleep difficulties are manifested in a variety of symptoms including, for example, the inability to fall asleep, interrupted sleep, insomnia, lack of restfulness, on and off sleep, and the tired, sluggish, or exhaustive feeling due to poor sleep and/or the lack of sleep. In order to alleviate symptoms related to sleep difficulties, there exist products such as sleeping pills and liquid formulations comprising a sleep agent.

Sleeping pills and liquid sleep formulations have been shown to provide some benefits to aid in the quality of normal sleep patterns. However, some sleeping pills and liquid sleep formulations can result in residual drowsiness, lethargy, hangover, tired, sluggish, and/or exhaustive feelings during wake hours. It is believed that these after-sleep or wake hours feelings are contributed to narcotic and/or hypnotic sleep agents that are contained in the sleeping pills or liquid sleep formulations. It has been shown that the incorporation of narcotic and/or hypnotic sleep agents can result in habituation, dependence, or addiction in addition to tolerance or withdrawal symptoms that can be associated with the sleeping pills or liquid sleep formulations containing such sleep agents.

Known sleeping pills such as melatonin have been shown to provide for normal sleep patterns. Melatonin can be administered orally, intranasally, and/or topically to treat symptoms of sleep difficulties. Melatonin, however, can be classified as a hypnotic sleep aid, thus the administration of melatonin can result in unwanted effects of feeling tired or sluggish during awakening hours.

Another example of sleeping aids that can provide for sleep benefits includes agents such as diphenhydramine and the benzodiazepenes. Diphenhydramine, however, is known as a sedative that has been shown to not only provide for effective treatment of symptoms related to sleep, but the administration of diphenhydramine can also result in a tired or sluggish feeling during awakening hours. The benzodiazepenes have been shown to have dependency and addictive properties.

Many parents of young children and their children suffer from the above mentioned sleep difficulties. Making sure you and your child are getting sufficient sleep is a critical aspect of child rearing. A recent National Sleep Foundation *Sleep in America* poll found that almost seven out of ten children (up to age 10) experience some type of sleep problem, and 75 percent of all parents want to change something about their child's sleep. See Sleeping Through the Night, J.Mindell, HarperResource (2005), pp. 3-5.

Part of the difficulty with getting infants and children to sleep is that their sleep is very different from adults. First, infants do not have the four stages of non-REM sleep seen in adults. Babies do not develop these distinct stages until about six months old. Another difference between adults' and infants' sleep is the organization of sleep. Infants sleep during many different periods during the day, whereas most adults consolidate all sleep to nighttime. As babies get older, they start to consolidate their sleep, taking less and less naps during the day.

Finally, another key difference between adults and infants is that most adults are able to easily get back to sleep if they wake during the night. As described by Mindell, "Waking during the night is a normal part of sleep. Many people though don't even know that they do it. They may awake for anywhere from a few seconds to a couple of minutes, and return immediately to sleep." In contrast to adults, many infants are unable to return to sleep on their own after waking during the night. They rely on help from adults to get them back to sleep and typically signal this need by crying. (Mindell, J. (2005). Sleeping Through the Night. *HarperResource,* pgs. 17-22) While disruptive to their parents, infant wakings during the night are to be expected up until about 6 months when "all babies are physically capable of sleeping through the night". ((Mindell, J. (2005). Sleeping Through the Night. *HarperResource,* pg. 26)

It is important is to note that these changes do not happen all at once, but rather over the period of time as the child develops to adolescence. During this time, there are specific needs for the child, as well as specific sleep tips for the parents or caregivers who is responsible for the care of the child. Interestingly, the National Sleep Foundation task force has specific recommendations for sleep as a child ages, breaking their recommendations and sleep tips into 5 distinct age groups: Infants (ages 2 months to 12 months), Toddlers (12 months to 3 years), Preschoolers (3 years to 6 years), School-aged (6 years to 12 years) and Adolescents (12 years to 18 years).

Unfortunately, for many families getting their baby to sleep through the night does not happen easily. Issues with sleep vary but the most common issues that parents face are night wakings, naptime problem, nighttime feedings, and bedtime struggles. Beyond being an aggravation to parents, sleep difficulties are a problem since getting enough sleep is imperative for healthy development. According to Dr. Mmindell. "babies who sleep through the night are better rested. happier. and less cranky during the day than babies who don't sleep. ((Mindell, J. (2005). Sleeping Through the Night. *HarperResource,* pg. 11) Beyond the benefit to the child. sleeping through the night is important to the well-being of the whole family since it enables parents to also get enough sleep and function better during the day.

Despite the great need, there are very few proven solutions to help parents ensure a good night's sleep for their children.

Many self-help books are available for parents to consult, such as, for example, Sleeping through the Night by Jodi Mitchell, 2005, pp 79-83 and Nighttime Parenting: How to Get Your Baby and Child to Sleep (La Leche League International Book) by William Sears (2005) pp. 37-43. Although these books suggest that parents instill good sleeping habits on their babies, for example by establishing a consistent bedtime routine, establishing a routine can be difficult, particularly if the infant is fussy, and parents may need more help than the book can offer. Additionally the routine and approaches recommended in the books can be effective in getting the infant to sleep but do not always succeed in helping the baby sleep through the night.

Furthermore, reading a book and trying to incorporate all the many recommendations is time consuming and a difficult task for many parents to incorporate into their already busy lives. Giving the child a bath is often recommended in these books as a way to help the baby calm down before sleep, however the recommendation fail to suggest the use of appropriate products and does not suggest the practice of a more comprehensive sensory regimen. Additionally, some recommend giving the child a bath only during the day since bathtime can be upsetting to some babies. The present invention overcomes this difficulty by offering specific combinations of products found to make the before bedtime experience positive.

In addition to books there are products available that claim to help infants sleep. For example, parents often give their children comforting toys to help them get to sleep, for example stuffed animals, dolls, blankets. Personal care products, such as Johnson's Bedtime Bath, offer parents a way to calm fussy babies, however these products may be limited in their utility since they only help calm the baby during the bath and may not be effective in improving other sleep behaviors specific to infants. Music products, such as Soothing Sounds for Sleep by Hush Little Baby and Baby Sleep by Erato are also available for parents to use before bed to help calm their infants.

For infants with clinically diagnosed sleep disorders, there are medications and devices, such as over-the-counter antihistamines, alpha-agonists, melatonin or herbal remedies, available that can help alleviate the disorders. However, due to the side effects associated with these therapies, they are not an appropriate solution for healthy infants with typical sleep difficulties.

The products and books available to help parents improve their infant's sleep behaviors are limited in their utility since they fail to both make bedtime easier and also help the infant sleep through the night. Additionally, many of the methods and products available are not pleasant or easy to follow. Establishing a consistent bedtime routine is so important in teaching infants to have positive sleep behaviors, yet many currently available products and methods fail to teach parents to incorporate them into a consistent routine. Thus there still remains a need for parents to have an effective way to establish a positive bedtime routine for their infants. Accordingly, there remains a need for a method for improving adult and infant sleep behaviors without relying on medications. The present invention answers this need.

### 3. SUMMARY OF THE INVENTION

It has been discovered that a two step regimen which incorporates a sensory fragrance in each step can be used to improve sleep behaviors of a human. Accordingly, the invention relates to a method for improving the sleep behaviors of a human comprising the administration of a sensory regimen which comprises:
a. the administration of a first sensory experience comprising topically applying to the skin or hair of the human a cleansing product containing an effective amount of a sensory fragrance; and
b. after step (a) the administration of a second sensory experience comprising administering an effective amount of a sensory fragrance.

The sensory fragrance used in step (a) and the sensory fragrance used in step (b) can be the same or different and is capable of reducing alpha activity.

The method according to the invention not only improves the sleep behaviors of adults but also surprisingly improves the sleep behaviors of a broad range of children under the age of eighteen, e.g. under the age of twelve and, in particular, infants, i.e., newborn children up to the age of three years old.

### 4. DETAILED DESCRIPTION OF THE INVENTION

As discussed above, the inventors have discovered a method for improving the sleep behaviors of a human which comprises the administration of a two step sensory regimen. The sensory regimen comprises
a. the administration of a first sensory experience comprising topically applying to the skin or hair of a human a cleansing product containing an effective amount of a sensory fragrance; and
b. after step (a) the administration of a second sensory experience comprising administering an effective amount of a sensory fragrance.

The sensory fragrance used in step (a) and the sensory fragrance used in step (b) can be the same or different and is capable of reducing alpha activity. In a particularly preferred embodiment, the sensory fragrance used in step (b) is the same as that used in step (a).

The cleansing step, i.e., step (a) is important because it is the initial introduction of the sensory fragrance, thus triggers the human to relax and initiates the association of the fragrance with a relaxing event. Preferably, the cleansing product application is done using warm water since the warm water is also relaxing and beneficial in promoting positive sleep behaviors since it is comforting and alters the human's body temperature. After the first sensory experience, i.e. the cleansing product application, a second sensory experience reinforces the promotion of relaxation and sleepiness as the administration of a sensory fragrance provides continued relaxing benefits. Preferably, the sensory fragrance used in the second sensory experience is the same sensory fragrance used in the first sensory experience since this builds an association between the two steps, thus enhancing the overall impact on sleep behaviors. The usage of a leave-on personal care product in the second step is particularly beneficial since the product containing the sensory fragrance stays on the skin beyond the duration of the actual sensory experience. For example, the usage of a skin moisturizing composition, e.g. a lotion, containing a sensory fragrance leaves the scent of the sensory fragrance on the human's skin so that it can be smelled throughout the night. This continued exposure to the scent of the sensory fragrance is particularly beneficial because it helps continue to relax the individual as they try to get to sleep and as they are sleeping through the night.

Suitable sensory fragrances include the perfume compositions described in co-pending U.S. Patent Application Publication No. US20040063604, the disclosure of which is hereby incorporated by reference. Such sensory fragrances aim to induce or be associated with positive, low activation moods and emotions. In one embodiment, a suitable sensory fragrance comprises from about 25% of at least five Relaxing Ingredients (R) selected from anethole, Bangalol.TM.. basil oil, cis-hex-3-enol, coumarin, ethylene brassylate, ethyl linalol, Florosa.TM., Galaxolide.TM., geraniol, cyclohexadecanolide, cyclopentadecanone, methyl anthranilate, alpha-iso-methyl ionone, Prunella.TM., Silvanone.TM., alpha-terpineol, Traseolide.TM., Ultravanil.TM., gamma-undecalactone, vetiver oil, vetiver acetate.

In one embodiment the sensory fragrance comprises:
(a) from about 25% in total of at least five (R); (b) optionally up to 45%, preferably up to 35%, more preferably up to 25%, in total of Non-Relaxing Ingredients (NR), provided that the ratio of R to NR is at least 0.75, preferably at least 0.9; (c) optionally up to 75% in total of Neutral Ingredients (N); (d) optionally up to 25% of other perfumery materials (M) provided that the weight ratio R to (M+NR) exceeds 0.75, preferably 0.9; and wherein:
   (i) all percentages are based on weight of the sensory fragrance; (ii) 'R' ingredients comprise anethole, Bangalol.TM., basil oil, cis-hex-3-enol, coumarin, ethylene brassylate, ethyl linalol, Florosa.TM., Galaxolide.TM., geraniol, cyclohexadecanolide, cyclopentadecanone, methyl anthranilate, alpha-iso-methyl ionone, Prunella.TM., Silvanone.TM., alpha-terpineol, Traseolide.TM., Ultravanil.TM., gamma-undecalactone, vetiver oil, vetiver acetate; (iii) 'NR' ingredients comprise methyl nonyl aldehyde (aldehyde MNA), allyl amyl glycolate, acetyl cedrene, Amberlyn Super.TM., amyl salicylate, armoise oil, benzyl salicylate, bergamot oil, Bourgeonal.TM., cedar leaf oil, citronellol, beta-damascone, dimethyl benzyl carbinyl acetate, Ethyl Safranate.TM., Everniate.TM., geranyl nitrile, Helional.TM., heliotropin, hexyl salicylate, lemon oil, Ligustral.TM., Lilial.TM., Lyral.TM., Mefrosol.TM., orange oil, orange terpenes, tagetes oil, tetrahydrogeraniol, vanillin; (iv) 'N' ingredients comprise benzyl acetate, cassis base, Cyclamen Aldehyde.TM., carvone, cinnarnic alcohol, dihydroeugenol, dihydromyrcenol, eugenol, Extralide.TM., galbanum, gamma-decalactone, hydroxycitronellal, indole, isoeugenol, jasmin oil, Jasmopyrane Forte.TM., linalol, linalyl acetate, methyl dihydrojasmonate (MDJ), octahydrocoumarin, patchouli oil. 2-phenylethyl alcohol, rose oxide, rose oil, Sandalone.TM., Sandalore.TM., styrallyl acetate, ylang-ylang; and (v) 'M' ingredients comprise perfumery materials not included in the above, excluding odourless or low-odour solvents or diluents used as vehicles for other perfume components.

Preferred sensory fragrances comprise at least 35% by weight in total of class R materials, even more preferably at least 45% by weight in total of class R materials. Also preferred are those perfumes wherein the weight ratio of R to NR (or R to the sum of NR and M) exceeds unity, or more preferably exceeds two, or is even higher. The sensory fragrance preferably includes optionally up to about 25% by weight in total of non-relaxing (NR) fragrance materials.

In a particularly preferred embodiment at least 5% by weight, or even more preferably at least 10% by weight, of the sensory fragrance comprises class R ingredients drawn from the following list, mostly characterized by exhibiting sweet and/or musky notes: coumarin, ethylene brassylate, Galaxolide.TM., cyclohexadecanolide, cyclopentadecanone, Traseolide.TM., Ultravanil.TM., gamma-undecalactone.

The sensory fragrance may optionally include one or more odourless or low-odour solvents and/or diluents, e.g. as a vehicle for a fragrance material. Any such solvents and/or diluents are not included when calculating percentages and ratios of R, NR, N and M materials of the composition.

Materials of class 'M' include prior art perfume materials which are not specified as being members of any of classes R, NR or N, excluding odourless or low-odour solvents or diluents, as noted above. They may be single ingredients, or mixtures both synthetic and natural (for example essential oils, and concretes).

As discussed above, the sensory fragrances useful in the present invention are created by combining a number of fragrance materials. The selection of these fragrance materials is based on extensive testing of fragrance materials, both by consumer testing and by measurement of brain electrical activity particularly alpha wave activity measured by electroencephalography (EEG), and statistical analysis of the resulting data to classify the materials into different categories, namely relaxing fragrance materials or ingredients (R) that induce in subjects exposed to them positive, low activation moods and emotions, such as relaxation (i.e. relaxing properties), non-relaxing fragrance materials or ingredients (NR) that induce in subjects exposed to them negative, high activation moods or emotions (i.e. non-relaxing properties), neutral fragrance materials or ingredients (N) having a neutral effect in terms of relaxing properties. Other fragrance materials (of which there are around three to four thousand currently available commercially and used in perfume formulation) are designated as class M materials. Based on this classification of fragrance materials, the invention enables sensory fragrances to be defined that are likely to induce in subjects exposed to them positive, low activation moods and emotions, such as relaxation. Such sensory fragrances are referred to herein for convenience as "sensory fragrances". The definition of the sensory fragrance nevertheless provides sufficient freedom in formulation to permit consideration of the hedonic properties of the composition. The invention can thus enable formulation of sensory fragrances that are relaxing and also have good hedonic properties.

In addition to the usage of the measurement of brain electrical activity to classify the fragrance materials used in creating the sensory fragrance, the measurement techniques are used in this invention to evaluate the final sensory fragrance. Therefore, a sensory fragrance according to this invention must contain the above-described fraction of relaxing ingredients and must also exhibit a reduction in alpha activity as measured using standard techniques. As previously mentioned, brain electrical activity particularly alpha wave activity can be measured by electroencephalography (EEG). Methods for using EEG are described on pages 110-121 and 149-153 of "Electroencephalography: Basic Principles, Clinical Applications, and Related Fields" (Niedermeyer, E. et al. 4th Edition, Williams & Wilkins, 1999). . Any method that can evaluate changes in alpha activity is acceptable for use in selecting an appropriate sensory fragrance for use in this invention. Because there are many methods of using EEG to evaluate alpha activity, the numerical magnitude of change necessary to be considered a true reduction will vary dependent on what method is used. The degree of alpha activity reduction necessary for the invention is that which is judged to be a significant change by a technician skilled in the art of using EEG.

As discussed above, the method according to the invention comprises the administration of two sensory experiences. In each step the sensory experiment may be continual. However, sensory experiences which are periodic are also useful in the present invention. For example, a suitable sensory experience would be smelling a fragranced stick for a few seconds at a time, on and off, for at least two minutes. Other appropriate periodic experiences could be relaxed breathing with the sensory fragrance being smelled at selected intervals of time, practicing yoga with the intermittent presentation of the sensory fragrance, or doing some form of exercise with periodic stops to smell a relaxing fragrance. Accordingly, the term "administering" refers to the (i) inhalation of a topically applied personal care composition containing a sensory fragrance; (ii) inhalation of the vapors which are released when a personal care composition containing a sensory fragrance is dissolved or dispersed in a liquid vehicle such as water, or (iii) inhalation of vapors which are released when a composition containing a sensory fragrance is dispersed, sprayed, melted or burned or otherwise released, for example, as in scented candles, air fresheners, and scented gels.

In the method according to the invention, the administration of the first sensory experience comprises topically applying to the skin or hair a cleansing product which contains an effective amount of a sensory fragrance. The sensory experience should be administered for an amount of time sufficient to provide the desired effect. In one embodiment, the first sensory experience is administered for at least five minutes. Generally, for infants the sensory experience should be administered for at least five minutes. When used on adults, i.e., humans of an age eighteen years or older, the first sensory experience is administered for at least ten minutes.

A cleansing product useful in the present invention may be prepared by blending an unfragranced wash base with a sensory fragrance. The two components should be combined using standard methods known to those skilled in the art of preparing cleansing product compositions. The compositions should be blended to ensure that the fragrance is evenly distributed, e.g., for at least 15 minutes. Optionally, the fragrance can be pre-blended with some of the base components in order to facilitate the blending process. Other cleaning product bases would also be suitable for combining with sensory fragrances.

The second sensory experience comprises the administration of a an effective sensory fragrance which can be delivered via a personal care product including cleansing and leave-on personal care products. Generally, the second sensory experience is administered for at least one minute, e.g., at least two minutes, e.g., at least five minutes.

In a particularly preferred embodiment, the secondary sensory experience comprises the administration of a leave-on personal care product, such as a moisturizer. The leave-on personal care product is preferably administered through massage. There are a number of different types of massages that can be used, and one is encouraged to use the technique that is comfortable and appropriate for the situation. Different massage types include, but are not limited to Acupressure, Deep Tissue Massage, Lomi Lomi, Lymphatic Drainage, Myofascial Release, Neuromuscular Therapy, Orthopedic Massage, Petrissage, Pregnancy Massage, Reflexology, Reiki, Rolfing, Sports Massage, Swedish Massage and Trigger Point massages. All of these massages can be used in conjunction with the application of the leave-on product to the skin.

Both cleansing and leave-on personal care products are particularly useful in delivering the sensory fragrance. For example, the sensory fragrance may be produced by blending the selected fragrance ingredients under ambient conditions until the final mixture is homogenous using equipment and methodology commonly known in the art. It is preferable to store the final sensory fragrance mixture under ambient conditions for a few hours after mixing before using it as a component of a cleansing or leave-on personal care product. In order to improve the solubilization of the sensory fragrance in aqueous personal care products, the sensory fragrance may be pre-blended with one or more of the nonionic surfactants.

Suitable cleansing personal care products include personal cosmetic, toiletry, and healthcare products such as dry and wet wipes, washes, baths, shampoos, gels, soaps, mousses, cleansing compositions, bath oils, other bath compositions that may be added to a bath, or other wash compositions that may be used directly on the skin. Any formulation useful for the above and which is compatible with the sensory fragrance is suitable for use in the present invention. In order to achieve the desired response in an infant mammal, the cleansing personal care product may be used in a dosing amount that is in accordance with the prescribed directions of the cleansing personal care product.

Suitable leave-on personal care products include personal cosmetic, toiletry, and healthcare products such as sticks, balms, mousses, sprays, lotions, creams, gels, powders, oils, waxes, perfumes, or other personal care compositions that may be applied to the skin and not rinsed off. Any formulation useful for the above and which is compatible with the sensory fragrance is suitable for use in the present invention. Methods for preparing suitable leave-on personal care products are well known to those skilled in the art of preparing personal care products. In order to achieve the desired response in an infant mammal, the leave-on personal care product may be used in a dosing amount that is in accordance with the prescribed directions of the cleansing personal care product.

Compositions containing the sensory fragrance and are capable of delivering the secondary sensory experience may also include, but are not limited to, wipes, washes, baths, shampoos, gels, soaps, sticks, balms, sachets, pillows, mousses, sprays, lotions, creams, cleansing compositions, oils, bath oils, aerosols, and substances which may be used with vaporizers.

The term "effective amount" refers to the amount of the sensory fragrance which is needed to create the desired response in a human of the desired age, for example, two months to three years old, two months to eighteen months, and two months to twelve months. Most importantly, the user must be able to perceive the odor of the sensory fragrance when administering the sensory experience according to its typical usage instructions. Preferably, the sensory fragrance will be used at a concentration greater than 0.05%. In one embodiment, the sensory fragrance is present in the cleansing product in an amount ranging from about 0.1 to about 1.5, for example 0.4 to 1.0, for example 0.4 to 0.6, percent by weight, based on the total weight of the cleansing product.

As discussed above, the present invention relates to a method for improving the sleep behaviors of a human. Examples of sleep behaviors that are improved include, for example, (a) perception of sleep quality; (b) a composite sleep quality score (c) perceived sleep depth; (d) number of night awakenings; (e) latency to sleep: (f) length of continuous period of sleep: (g) ratio of amount of night time to amount of day time sleep; (h) amount of time before falling asleep; (i) amount of time during the night spent awake; (j) amount of time during the night spent sleeping; (k)amount of time during the day spent sleeping; (I) mood following sleep; (m) degree of pre-sleep fussiness; (n) satisfaction with sleep; (o) occurrence of early awakenings, (p) difficulty going to sleep; (q) morning alertness; and (r) combinations thereof. Obviously, certain sleep behaviors are more relevant which assessing infant sleep as opposed to adult sleep.

Sleep behaviors can be assessed through a number of different tools including for example, a questionnaire; an interview; a psychometric assessment tool; a biometric assessment tool; or any combinations thereof. For example, perception of sleep quality and quantity can be assessed using simple questions, like "How long do you typically sleep at night?" or "Do you have any difficulties sleeping?". However, it is preferable to use one of the many available sleep psychometric tools for assessing additional sleep-related behaviors such as length of continuous period of sleep, degree of pre-sleep fussiness, mood state after morning awakening, ratio of amount of night time to amount of day time sleep, number of night awakenings, time before falling asleep, amount of time during the night spent awake, amount of time during the night spent sleeping, amount of time during the day spent sleeping, mood following sleep, and composite sleep quality. These can be measured by different psychometric tools for assessing sleep related behaviors, including but not limited to, The St. Mary's Hospital Sleep Questionnaire (Sleep. 1988 Oct;11(5):448-53.), the Leeds Sleep Evaluation Questionnaire (Hum Psychopharmacol. 2003 Dec;18(8):603-10.), and the Pittsburgh Sleep Quality Index (Journal of Psychosomatic Research, 45(1), 5-13), and the Profile of Mood States (POMS) (McNair, D. M., Lorr, M., & Droppleman, L. F. (1971). Manual for the Profile of Mood States (POMS). San Diego, CA: Educational and Industrial Testing Service). Biometric assessments such as actigraphy and polysomnography, Life Vest by Vivometrics, devices by BodyMedia, a standard thermometer, a core temperature thermometer, a seismometer, a Biosensor by Exmover R are also useful in assessing sleep.

Parents of children who do not sleep well also do not sleep well and suffer mood problems. Accordingly, in another embodiment, the invention relates to a method for improving the mood state of a parent of a human child of an age ranging from two months to six years old, said method comprising the administration of a sensory regimen to said child wherein said regimen comprises:
a. the administration of a first sensory experience comprising topically applying to the skin of said human a cleansing product containing an effective amount of a sensory fragrance as described above; and
b. after step (a) the administration of a second sensory experience comprising administering an effective amount of a sensory fragrance as described above, wherein the sensory fragrance used in step (a) and said sensory fragrance used in step (b) is the same or different.

Parents of children between the ages of two and eighteen months are generally expected to see the most benefit from the above method. As used herein "parent" means a caregiver of an infant or child including but not limited to, the infant or child's mother or father, grandparent, guardian or babysitter.

In another embodiment, the invention relates to a kit comprising (a) a cleansing product containing an effective amount of a sensory fragrance as described above and (b) a personal care product, which is other than a cleansing product and which contains an effective amount of a sensory fragrance as described above. The sensory fragrance used in the cleansing product and the sensory fragrance used the personal care product may be the same or different. The kit may be packaged by shrink wrap material, in a box, in a basket, in a bag or virtually bundled, for example, offered for sale on the Internet, in a coupon or other promotional material, as a combination product containing the cleansing product and the personal care product.

The method of the present invention includes the administration of compositions that can comprise, consist of, or consist essentially of the elements and limitations described herein, as well as any of the additional or optional ingredients, components, or limitations described herein.

The following examples further describe and demonstrate embodiments within the scope of the present invention. The examples are given solely for the purpose of illustration and are not to be construed as limitations of the present invention, as many variations thereof are possible without departing from the spirit and scope of the invention.

### EXAMPLES

### Example 1. Sensory Fragrances

Examples of sensory fragrances useful in the present invention are described in Table 1. These fragrances are unique in their ability to reduce alpha activity. All contain at least 25% of relaxing ingredients. The fragrances below and others like them are readily available from Quest International.

Not all pleasant smelling fragrances are appropriate for use as a sensory fragrance because not all pleasant fragrances are able to incur a reduction in Alpha activity nor do all pleasant fragrances contain at least 25% relaxing ingredients.

**Table 1: Examples of Sensory Fragrances**

| **Fragrance Name** | **Shown to cause a reduction in Alpha activity?** | **Contains at least 25% relaxing ingredients?** |
|---|---|---|
| Crème d'Amour | Yes | Yes |
| Pastel Petals | Yes | Yes |
| Tender Touch | Yes | Yes |
| Pink Pashmina | Yes | Yes |

### Example 2. Cleansing Product Containing Sensory Fragrance

A cleansing product useful in the present invention and described in Table 2 below was prepared by blending unfragranced Johnson's Head-to-Toe wash base with sensory fragrance Pastel Petals available from Quest International. The two components were combined using standard methods known to those skilled in the art of preparing cleansing product compositions. The wash base and sensory fragrance composition was blended for at least 15 minutes to ensure that the fragrance was evenly distributed.

**Table 2: Formulation for Cleansing Product Containing Sensory Fragrance**

| **Ingredient** | **Percentage** |
|---|---|
| Unfragranced Johnson's Head-to-Toe Wash base | 99.5% |
| Pastel Petals (or other sensory fragrance) | 0.5% |

### Example 3: Skin Moisturizing Composition Containing Sensory Fragrance

A skin moisturizing composition useful in the present invention and described in Table 3 below was prepared by blending unfragranced Johnson's 24-hour Soft Lotion base with Pastel Petals fragrance available from Quest International. The two components were combined using standard methods known to those skilled in the art of preparing skin moisturizing compositions. The composition was blended for at least 15 minutes to ensure that the fragrance was evenly distributed.

**Table 3: Formulation for Skin Moisturizing Composition Containing Sensory Fragrance**

| Ingredient | Percentage |
|---|---|
| Unfragranced Johnson's 24-Hour Soft Lotion base | 99.5% |
| Pastel Petals (or other sensory fragrance) | 0.5% |

### Example 4. Adult Sleep Study Results

A group of healthy, adult women was recruited to participate in a study to assess the sleep behavior benefits of following a sensory regimen every night for two weeks. The women were divided into two treatment groups with 22 women per group.

Group A followed a regimen comprising the usage of the cleansing product of Example 2, referred to as Bath/Wash Product below, and the skin moisturizing composition of Example 3, referred to as Massage/Lotion Product below, every night at bedtime. The specific regimen followed by Group A is shown in Table 4.

Group B followed a sensory regimen comprising the usage of the cleansing product of Example 2, referred to as Bath/Wash Product below, and the skin moisturizing composition of Example 3, referred to as Massage/Lotion Product below, relaxing music and dimmed lighting every night at bedtime. The specific regimen followed by Group B is shown in Table 5.

**Table 4: Group A's Prescribed Sensory Regimen**

| **Adult Sleep Sensory Regimen - Group A** | |
|---|---|
| Please complete the following regimen in the evening, preferably just before going to bed. | |
| **1. Bath** | - Start your bath, adjusting the water to a comfortably warm temperature. - Add 3-4 capfuls of the provided **Bath/Wash Product** to the running water. *- Optional:* Use the provided poof to wash during the bath - Relax and bathe for a total of about 10 minutes or longer |
| **2. Self-Massage** | - After drying, find a comfortable spot to sit (either in the bathroom or another room) - Dispense the **Massage/Lotion Product** into the palm of your hand. Use as much lotion as you feel necessary and dispense as frequently as you want. - Slowly, gently but firmly massage the lotion into your arms, shoulders, hands, thighs, knees, calves, feet and soles of feet. - Your self-massage should last about 5 minutes or longer |
| **3. Bedtime** | Please go to bed as soon as possible after completing the regimen. If you can't go directly to bed, please avoid strenuous or stressful activities and only do relaxing activities (watch TV, read, etc.) before you go to bed. |

**Table 5: Group B's Prescribed Sensory Regimen**

| **Adult Sleep Sensory Regimen - Group B** | |
|---|---|
| Please complete the following regimen in the evening, preferably just before going to bed. | |
| **1. Bath** | - Prepare the bathroom for your bath by dimming the lights and playing your relaxing music. Use the provided click light and relaxing music if you don't have your own. |
| | - Start your bath, adjusting the water to a comfortably warm temperature. |
| | - Add 3-4 capfuls of the provided **Bath/Wash** Product to the running water. |
| | *- Optional:* Use the provided poof to wash during the bath |
| | - Relax and bathe for a total of about 10 minutes or longer |
| **2. Self-Massage** | - After drying, find a comfortable spot to sit (either in the bathroom or another room). Bring your relaxing music with you if possible. |
| | - Dispense the **Massage/Lotion** Product into the palm of your hand. Use as much lotion as you feel necessary and dispense as frequently as you want. |
| | - Slowly, gently but firmly massage the lotion into your arms, shoulders, hands, thighs, knees, calves, feet and soles of feet. - Your self-massage should last about 5 minutes or longer |
| **3. Bedtime** | Please go to bed as soon as possible after completing the regimen. If you can't go directly to bed, please avoid strenuous or stressful activities and only do relaxing activities (watch TV, read, etc.) before you go to bed. |

Subjects in Group B were asked to select music that they found relaxing. The dimmed lighting used by Group B was made by either using a dimmer switch or by using unscented candles or a battery operated closet light in place of the bathroom's overhead light. All subjects were also given a bathrobe to use to keep warm after the bath and while applying the skin moisturizing composition.

The duration of the study was three weeks. During the first week, i.e. the Baseline week, subjects completed the questionnaires but followed their normal sleep routine, i.e. they did not follow the sensory regimen. During Treatment Weeks 1 and 2 subjects followed their assigned sensory regimen every night. Multiple self-report questionnaires were completed by the subjects throughout the study in order to assess the impact of following the sensory regimens on their mood and sleep behaviors. A summary of the study design, measures and frequency of the measures is shown in Table 6.

**Table 6. Adult Sleep Study Design, Measures, and Frequency of Measures**

| **Week** | **Subject Instructions** | **Measures** |
|---|---|---|
| **Baseline** | Follow your normal before bed routines and complete the | - St. Mary's Sleep Questionnaire (3 x per week) |
| | questionnaires provided | - Profile of Mood States (before and after typical bedtime routine, 1x per week) |
| **Treatment Week 1** | Complete your assigned sensory regimen every night before bed | - St. Mary's Sleep Questionnaire (3 x per week) |
| | (either Group A or B) and complete the questionnaires provided | - Profile of Mood States (before and after sensory regimen, 1x per week) |
| **Treatment Week 2** | Complete your assigned sensory regimen every night before bed | - St. Mary's Sleep Questionnaire (3 x per week) |
| | (either Group A or B) and complete the questionnaires provided | - Profile of Mood States (before and after sensory regimen, 1x per week) |

To assess improvements in sleep quality the panelists were asked to complete the St. Mary's Sleep Questionaire. The St. Mary's Sleep Questionnaire, as described by T. J. Leigh, H. A. Bird, I. Hindmarch, P. D. Constable. V. Wright in "Factor analysis of the St. Mary's Hospital sleep questionnaire" Sleep 1988; 11: 448-453, is a self-assessment questionnaire used in the field of sleep research to quantitatively evaluate a range of sleep parameters. The questions and rating scales used in this questionnaire are as follows:

This questionnaire refers to your sleep over the past 24 hours. Please try to answer every question.

At what time did you:

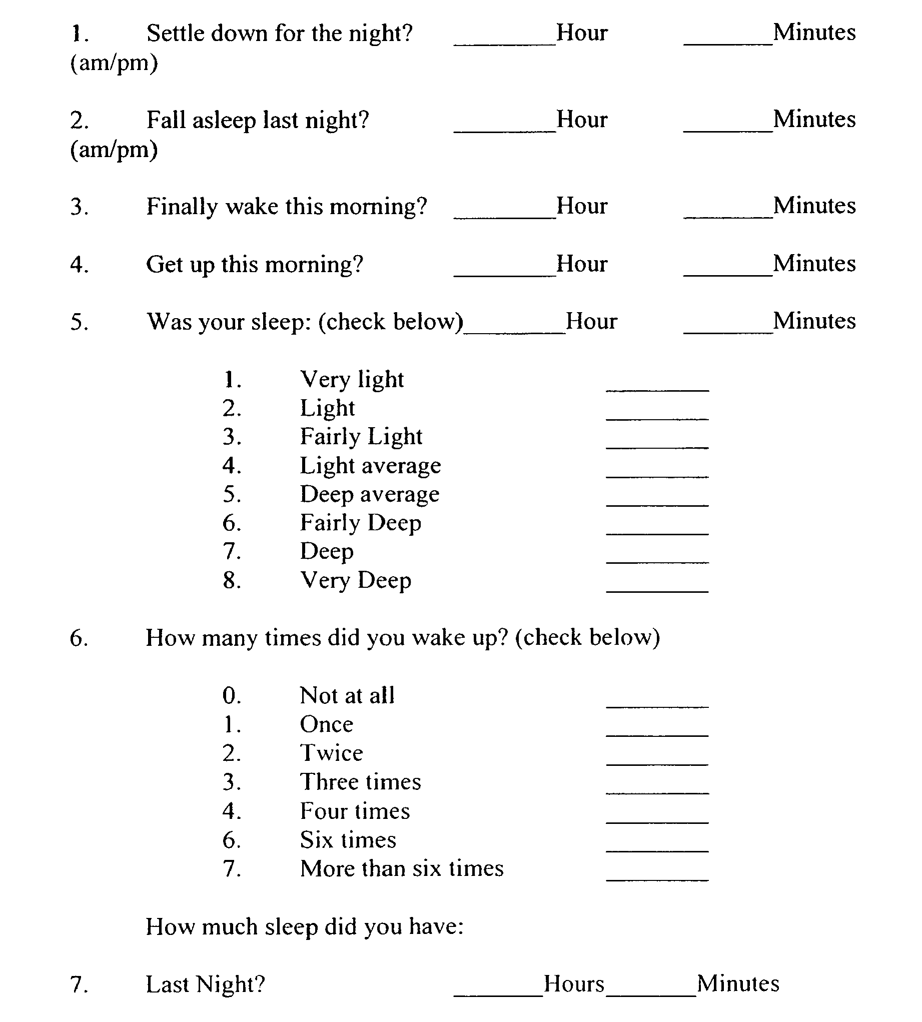

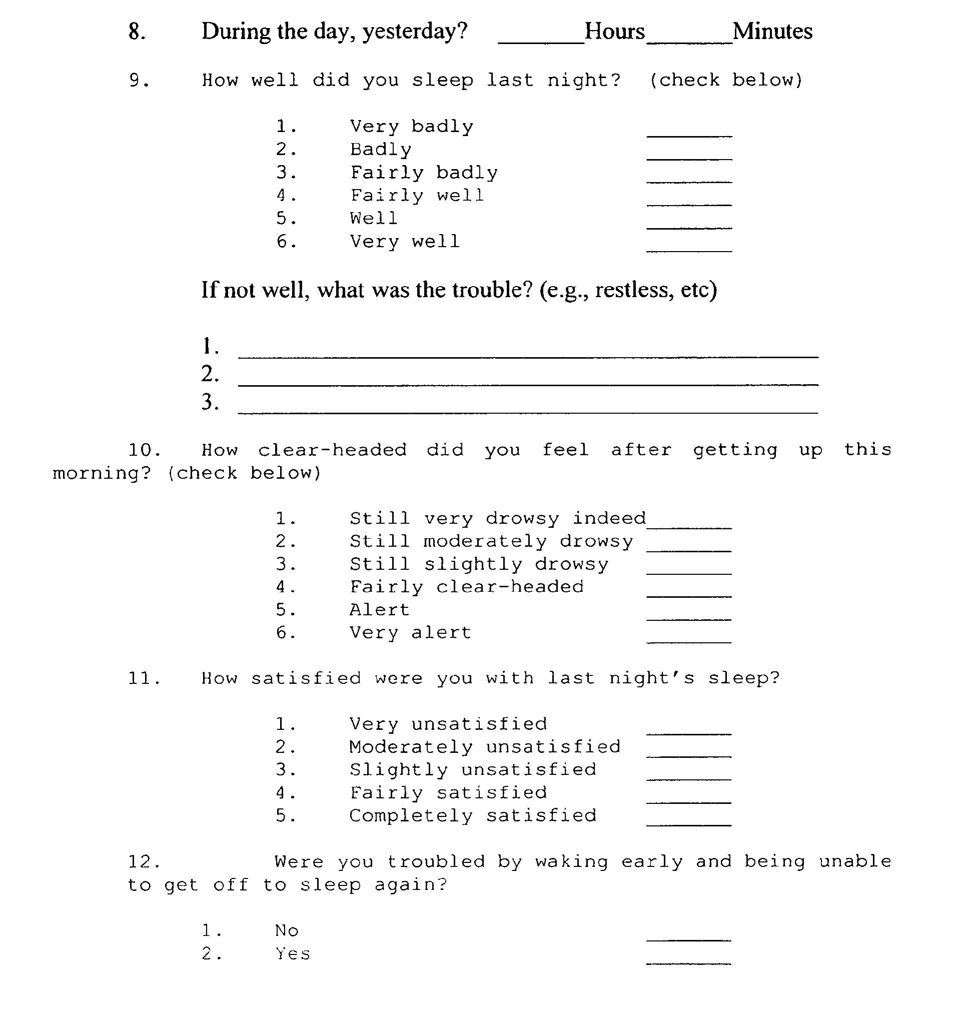

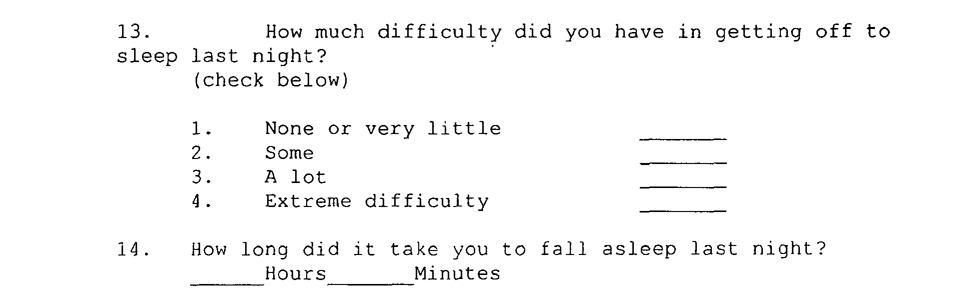

Panelists rated each parameter on the given scale accordingly. A composite sleep quality score was calculated according to the following formula which uses the subject's numerical responses to the questions detailed above: (Question #10) + (Question #5) + (Question #11) + (Question #9) - [(Question #13)+(0.1 x (Question #14)) + (Question #6) + (Question #12)]. A greater composite sleep quality score is indicative of better quality sleep. Other parameters were assessed using data from the individual questions in the questionnaire. The questionnaire was completed three times a week during each week of the three-week study, resulting in three sets of sleep parameters each week. The three sets of data were averaged resulting in a score on each parameter for each week of the study, i.e. Baseline, Treatment Week 1, and Treatment Week 2. In order to evaluate the changes in sleep behavior, percent differences were calculated to show the change in each parameter in Treatment Week 1 and Treatment Week 2 versus Baseline. The results for Groups A and B are shown in Table 7 below.

**Table 7: Results from the St. Mary's Sleep Questionnaire**

| **Parameter** | **% Change: Baseline vs. Treatment Week 1** | | **% Change: Baseline vs. Treatment Week 2** | |
|---|---|---|---|---|
| | **Group A** | **Group B** | **Group A** | **Group B** |
| Composite sleep quality score* | +23% | +3% | +44% | +30% |
| Perceived Sleep Depth (Question #5) | +10% | +2% | +18% | +8% |
| Number of Night Awakenings (Question #6) | -28% | +4% | -50% | -29% |
| Latency to Sleep (Question #14) | -4% | +6% | -33% | -27% |
| Satisfaction (Question #11) | +9% | +5% | +17% | +9% |
| Occurrence of Early Awakenings (Question #12) | +3% | +3% | +3% | -1% |
| Difficulty Going to Sleep (Question #13) | -6% | -5% | -16% | -16% |
| Morning Alertness (Question #10) | +3% | +7% | +16% | +12% |
| Amount of Time During the Night Spent Sleeping (Question #7) | -1% | -1% | +3% | +3% |

| | | | | |
|---|---|---|---|---|
| * Composite sleep quality score = (Question #10) + (Question #5) + (Question #11) + (Question #9) - [(Question #13)+(0.1 x (Question #14)) + (Question #6) + (Question #12)]. All questions refer to the St. Mary's Sleep Questionnaire as detailed above. | | | | |

In order to assess the impact of the sensory regimens on the subjects' moods, subjects were also asked to complete the Profile of Mood States (POMS). The POMS was originally published by McNair. Lorr and Droppleman in 1971. (McNair, D. M., Lorr, M., & Droppleman, L. F. (1971). Manual for the Profile of Mood States (POMS). San Diego, CA: Educational and Industrial Testing Service.) The POMS identifies and assesses transient, fluctuating affective mood states in psychiatric outpatients. The POMS has been proven to be particularly useful in measuring change in mood states over time. In particular, the POMS examines the mood states of Tension-Anxiety, Depression-Dejection, Anger-Hostility, Vigor-Activity, Fatigue-Inertia, Confusion-Bewilderment. Scores for each of these mood states are calculated by adding subjects' responses to which of five possible answers (0 = Not at all, 1 = A little, 2 = Moderately, 3 = Quite a bit, and 4 = Extremely) best describes "how they have been feeling". The scale comprises 65 total items which are grouped according to Table 8 in order to establish scores for each of the indicated mood states. In this study, the POMS was completed before and after the sensory regimen in order to evaluate the change in mood resulting from following the regimen. Percent changes in the mood scores were calculated for each of the two Treatment Weeks and the resulting percentages were averages resulting in the data shown below in Table 9.

**Table 8. Summary of POMS Mood Scales and Items**

| **Mood Scale** | **Items (-) indicates reverse scoring** |
|---|---|
| | Tense |
| | Shaky |
| | On edge |
| | Panicky |
| Tension-Anxiety | Relaxed (-) |
| | Uneasy |
| | Restless |
| | Nervous |
| | Anxious |
| | Angry |
| | Peeved |
| | Grouchy |
| | Spiteful |
| | Annoyed |
| Anger-Hostility | Resentful |
| | Bitter |
| | Ready to fight |
| | Rebellious |
| | Deceived |
| | Furious |
| | Bad-tempered |
| | Worn out |
| | Listless |
| | Fatigued |
| Fatigue | Exhausted |
| | Sluggish |
| | Weary |
| | Bushed |
| | Unhappy |
| | Sorry for things done |
| | Sad |
| | Blue |
| Depression - Dejection | Hopeless |
| | Unworthy |
| | Discouraged |
| | Lonely |
| | Miserable |
| | Gloomy |
| | Desperate |
| | Helpless |
| | Worthless |
| | Terrified |
| | Guilty |
| | Lively |
| | Active |
| | Energetic |
| | Cheerful |
| Vigor | Alert |
| | Full of pep |
| | Carefree |
| | Vigorous |
| | Confused |
| | Unable to concentrate |
| | Muddled |
| Confusion-Bewilderment | Bewildered |
| | Efficient (-) |
| | Forgetful |
| | Uncertain about things |

**Table 9: Results of Profile of Mood States**

| **Mood Scale** | **Average Change Treatment Weeks 1 & 2 (After-Before Sensory Regimen)** | |
|---|---|---|
| | **Group A** | **Group B** |
| **Tension - Anxiety** | -54% | -63% |
| **Depression-Dejection** | -47% | -51% |
| **Anger-Hostility** | -74% | -69% |
| **Fatigue** | -19% | -24% |
| **Vigor** | -12% | -20% |
| **Confusion-Bewilderment** | -7% | -4% |

As shown in Tables 7 and 9, both of the regimens, i.e. the regimen used by Group A and the regimen used by Group B were effecting in improving both the sleep behaviors and mood states of the healthy women who participated in the study. In particular, as indicated by the significant increases in composite sleep quality score and improvements in the individual parameters, the regimens were extremely effective in improving many sleep behaviors including composite sleep quality score, perceived sleep depth, number of night awakenings, latency to sleep, satisfaction with sleep, occurrence of early awakenings, difficulty going to sleep, morning alertness. Furthermore, both sensory regimens were effective in improving multiple mood states including the reduction of tension-anxiety, anger-hostility, depression-dejection, fatigue, and confusion-bewilderment.

### Example 5. Infant Sleep Study

A group of 58 healthy infants, ages 7 months to 18 months, and their parents were recruited to participate in a study to assess the sleep behavior benefits of following a sensory regimen every night for two weeks.

The subjects followed a bedtime sensory regimen comprising the usage of the cleansing product of Example 2, referred to as Bath/Wash Product below, and the skin moisturizing composition of Example 3, referred to as Massage/Lotion Product below, every night at bedtime. The specific bedtime sensory regimen is shown in Table 10.

**Table 10: Infant Bedtime Sensory Regimen**

| **Infant Bedtime Sensory Regimen** | |
|---|---|
| Please begin this Bedtime Sensory Regimen at a time that will allow you to put your baby down for bed within 30 minutes after the bath. | |
| **1. Bath** | Please use the **Bath/Wash Product** as follows: |
| (To be done in your normal | 1.Fill full size tub with warm water to the tub fill level and temperature which you are comfortable with. |
| bathing | 2.Pour 2 - 4 capfuls of product into warm bath water. |
| environment) | 3.Swirl product with hand to mix evenly. |
| | 4.For cleansing, use only the tub water (with product already dispersed) on a wet washcloth or with hands. |
| | 5.Rinse off product after cleansing |
| | 6.Dry baby as you normally do after bathing. |
| | |
| | Bathe your baby for a minimum of 5 minutes and a maximum of 10 minutes. |
| **2. Dry /Lotion** / **Dress** | After drying your baby, please use the **Massage/Lotion Product** as follows: |
| | 1. Dispense product into palm of hand |
| | 2. Massage gently into skin, applying to feet, legs, thighs, tummy, arms hands, torso etc. for a minimum of 2 minutes up to a maximum of 5 minutes. |
| | |
| | Diaper and dress your baby for bed at your discretion. |
| **3. After the Bath** | After the bath, please do whatever else you normally do just before putting your baby down for the night. This can include quiet activities such as soft music, reading books, singing lullabies, feeding, etc. No active play including television should take place after the bath. Please put your baby down for bed no more than 30 minutes after finishing the bath! |

The duration of the study was three weeks. During the first week, i.e. the Baseline week, the parents of the infant subjects completed questionnaires but followed their infant's typical bedtime routine, i.e. they did not follow the sensory regimen. During Treatment Weeks 1 and 2 the parents followed the bedtime sensory regimen with their infants every night. The parents completed multiple questionnaires throughout the study in order to assess the impact of following the sensory regimens on their mood and their infants' sleep behaviors. A summary of the study design, measures and frequency of the measures is shown in Table 11.

**Table 11. Infant Sleep Study Design, Measures, and Frequency of Measures**

| **Week** | Subject Instructions | Measures |
|---|---|---|
| **Baseline** | Follow your infant's normal before bedtime routine and | - Brief Infant Sleep Questionnaire (1x per week) |
| | complete the questionnaires provided | - Profile of Mood States (1x per week) |
| | | - Daily Sleep Diary (7x per week) |
| **Treatment Week 1** | Complete the sensory regimen with your infant every night | - Brief Infant Sleep Questionnaire (1× per week) |
| | before bed and complete the questionnaires provided | - Profile of Mood States (1× per week) |
| | | - Daily Sleep Diary (7x per week) |
| **Treatment Week 2** | Complete the sensory regimen with your infant every night | - Brief Infant Sleep Questionnaire (1x per week) |
| | before bed and complete the questionnaires provided | - Profile of Mood States (1x per week) |
| | | - Daily Sleep Diary (7x per week) |

To assess improvements in their infants' sleep, parents were asked to complete the Brief Infant Sleep Questionnaire. The Brief Infant Sleep Questionnaire, as described in "A Brief Screening Questionnaire for Infant Sleep Problems: Validation and Findings for an Internet Sample (A. Sadeh, Pediatrics, Vol. 113 No. 6 June 2004) is a validated psychometric tool used in the field of infant sleep research to quantitatively evaluate a range of infant sleep parameters. The questions and rating scales used in this questionnaire are as follows:

### Brief Infant Sleep Questionnaire (BISQ)

Please think about your baby's sleep, on average, over the past seven nights in answering the following questions.

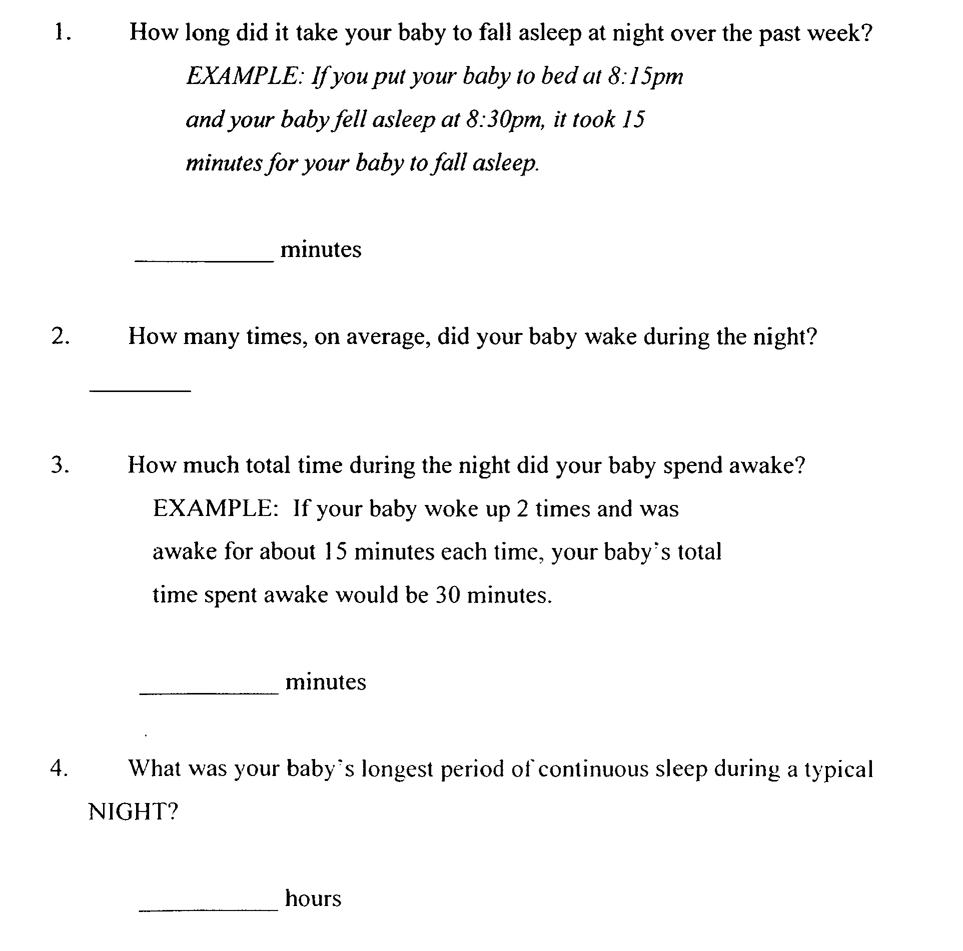

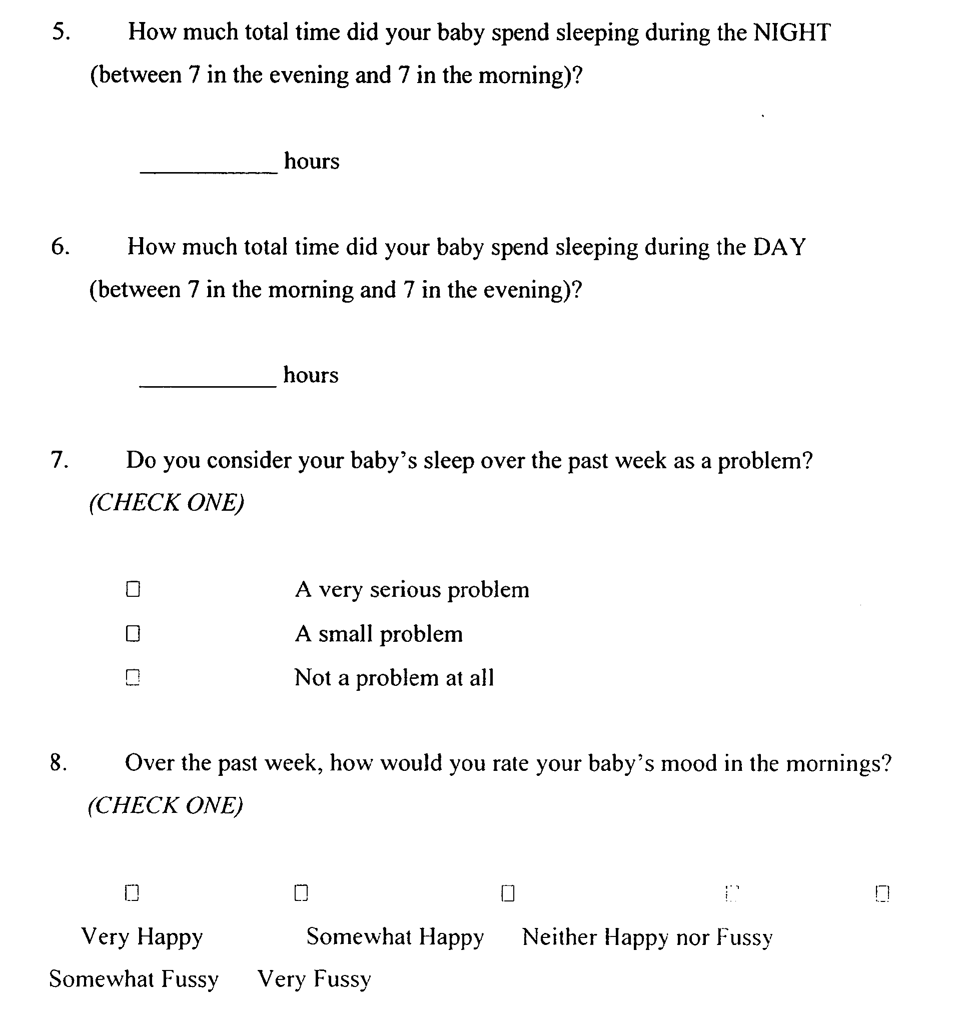

Panelists rated each parameter on the given scale accordingly. The results from the Brief Infant Sleep Questionnaire are shown in Table 12 below.

**Table 12: Results of Brief Infant Sleep Questionnaire**

| **Parameter** | **BISQ Question** | **Baseline** | **End of Treatment Week 1** | **End of Treatment Week 2** |
|---|---|---|---|---|
| **Amount of time before falling asleep** | 1. How long did it take your baby to fall asleep? (in minutes) | 22.04 | 15.16 | 13.83 |
| **Number of night awakenings** | 2. How many times, on average, did your baby wake during the night? | 2.07 | 1.47 | 1.29 |
| **Amount of time during the night spent awake** | 3. How much total time during the night did your baby spend awake? (in minutes) | 36.2 | 20.79 | 18.45 |
| **Length of continuous period of sleep** | 4. What was your baby's longest period of continuous sleep during a typical night? (in hours) | 7.12 | 8.33 | 8.78 |
| **Amount of time during the night spent sleeping** | 5. How much total time did your baby spend sleeping during the night? (in hours) | 9.49 | 10.05 | 10.3 |
| **Amount of time during the day spent sleeping** | 6. How much total time did your baby spend sleeping during the day? (in hours) | 3.21 | 3.17 | 3.24 |
| **Perception of sleep quality** | 7. Do you consider your baby's sleep over the past week as a problem? (1 = A very serious problem, 2 = A small problem, 3 = Not a problem at all) | 2.02 | 2.6 | 2.74 |
| **Mood state following sleep** | 8. Over the past week, how would you rate your baby's mood in the morning? (5 = Very Happy, 4 = Somewhat Happy, 3 = Neither Happy Nor Fussy, 2 = Somewhat Fussy, 1 = Very Fussy) | 3.78 | 4.26 | 4.47 |

In order to further assess improvements in their infants' sleep, parents were asked to complete a Daily Sleep Diary. The diary was based on the same questions asked in the previously described Brief Infant Sleep Questionnaire but was adapted for daily use. Additional questions were also added to evaluate sleep behaviors not included in the Brief Infant Sleep Questionnaire.

The Daily Sleep Diary as used in the study is shown here:

### Daily Sleep Diary

Please complete this diary every MORNING of the study. Please think about your baby's sleep during the previous day and night in answering the following questions.

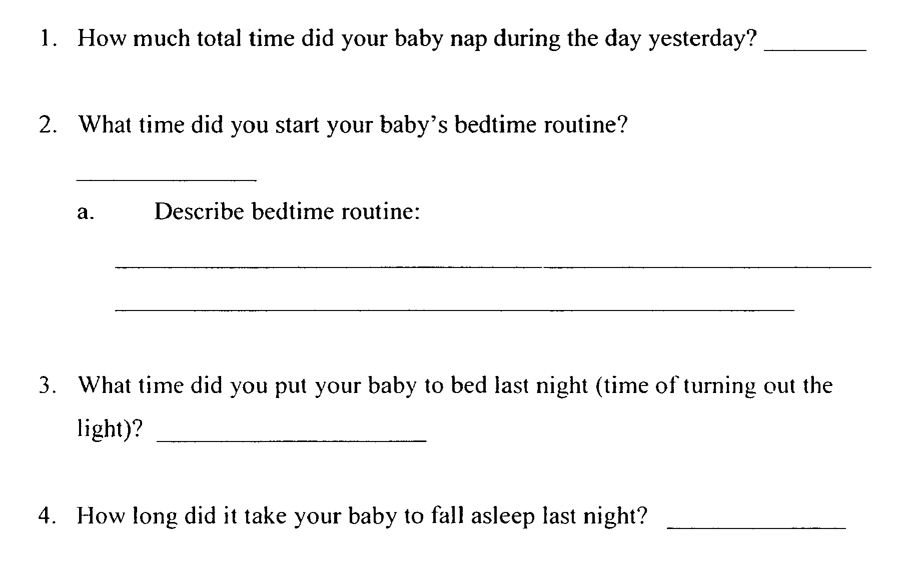

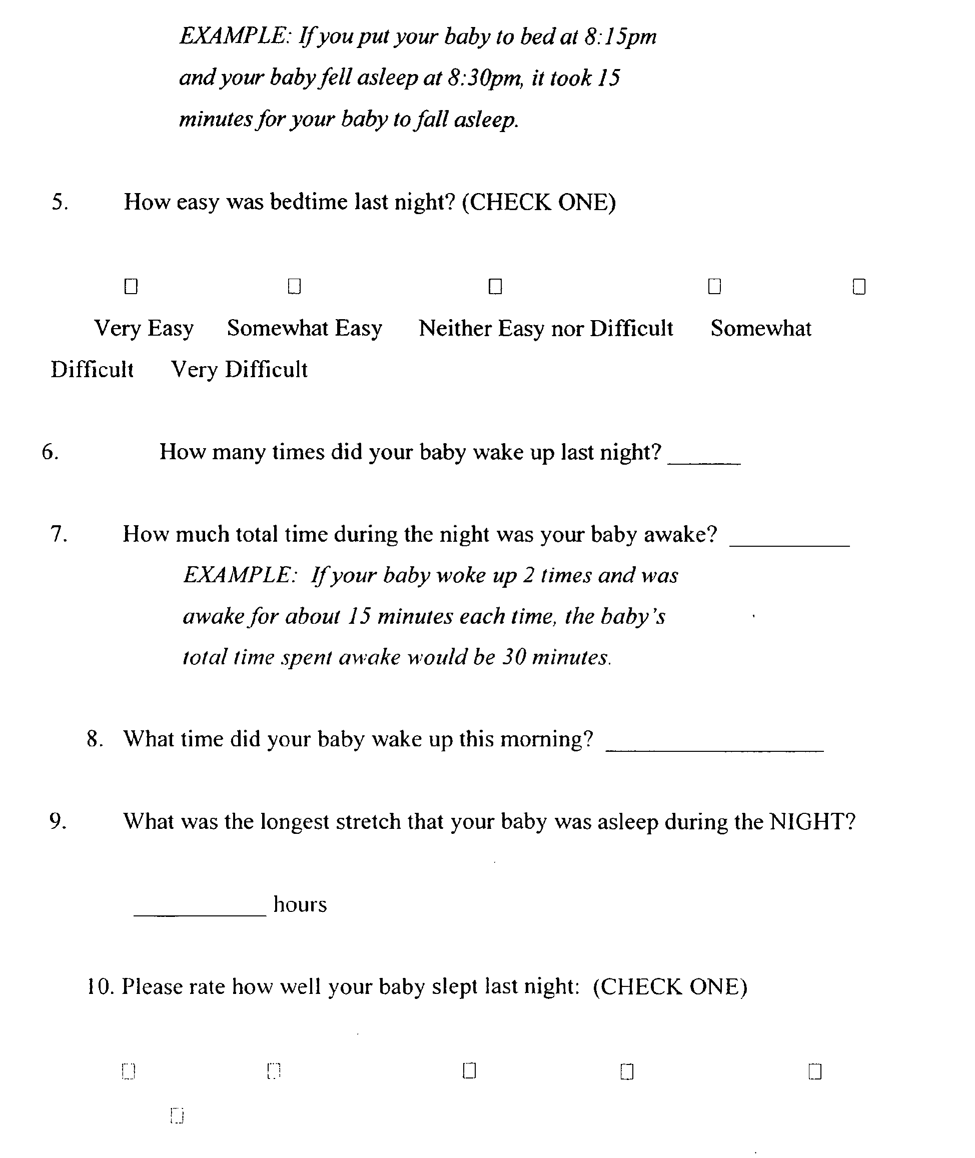

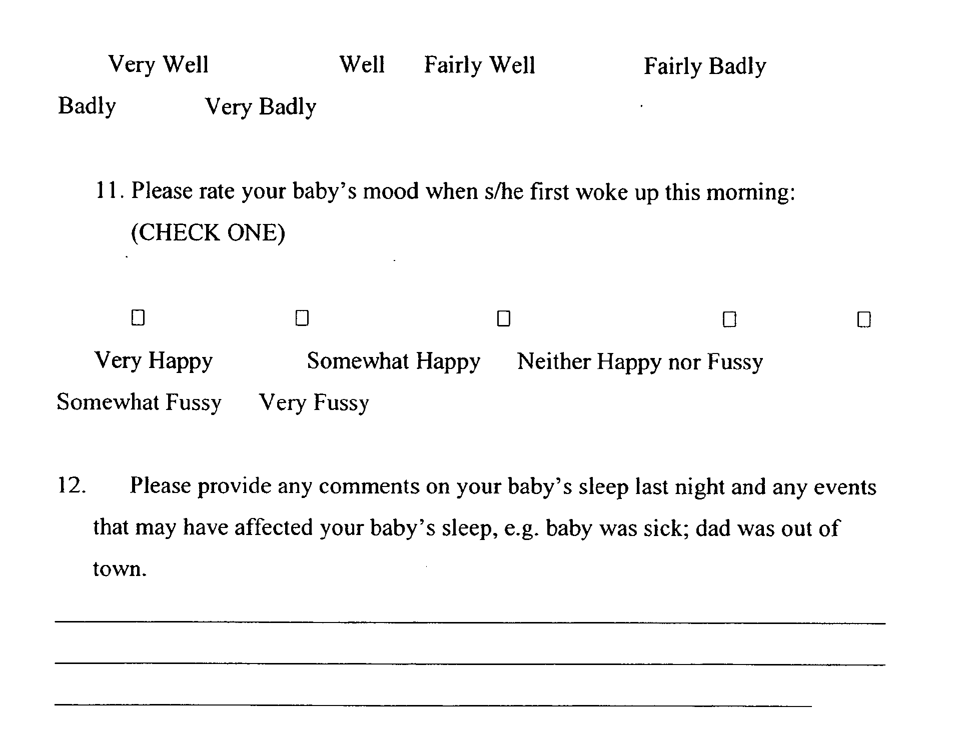

Panelists rated each parameter on the given scale accordingly. Responses to each question were averaged across each week, i.e. the average comprised seven days of data, resulting in an average response for the three study weeks: Baseline, Treatment Week 1. and Treatment Week 2. Pertinent weekly average results from the Daily Sleep Diary are shown in Table 13 below.

**Table 13: Weekly Average Results from the Daily Sleep Diary**

| **Parameter** | **Daily Sleep Diary Question** | **Baseline Average** | **Treatment Week 1** | **Treatment Week 2 Average** |
|---|---|---|---|---|
| **Amount of time during the day spent sleeping** | 1. How much total time did your baby nap during the day yesterday? (in hours) | 2.46 | 2.47 | 2.53 |
| **Amount of time before falling sleep** | 4. How long did it take your baby to get to bed last night? (in minutes) | 21.17 | 14.36 | 12.52 |
| **Degree of pre-sleep fussiness** | 5. How easy was bedtime last night? (5 = Very Easy, 4 = Somewhat Easy, 3 = Neither Easy Nor Difficult, 2 = Somewhat Difficult, 1 = Very Difficult) | 3.56 | 4.16 | 4.38 |
| **Number of night awakenings** | 6. How many times did your baby wake up last night? | 1.87 | 1.4 | 1.10 |
| **Amount of time during the night spent awake** | 7. How much total time during the night was your baby awake? (in minutes) | 31.98 | 17.86 | 14.88 |
| **Length of continuous period of sleep** | 9. What was the longest stretch that your baby was asleep during the night? (in hours) | 6.02 | 7.35 | 8.03 |
| **Perception of sleep quality** | 10. Please rate how well your baby slept last night. (6 = Very Well, 5 = Well, 4 = Fairly Well, 3 = Fairly Badly, 2 = Badly, 1 = Very Badly) | 4.19 | 4.97 | 5.30 |
| **Mood state following sleep** | 11. Please rate your baby's mood when s/he first woke up this morning. (5 = Very Happy, 4 = Somewhat Happy, 3 = Neither Happy Nor Fussy, 2 = Somewhat Fussy, 1 = Very Fussy) | 3.69 | 4.08 | 4.33 |

In order to assess the impact of the sensory regimens on the parents' moods, the parents were also asked to complete the Profile of Mood States (POMS). Details on the Profile of Mood States can be found in Example 4. The scale comprises 65 total items which are grouped according to Table 8 in order to establish scores for each of the indicated mood states.

In this study, the POMS was completed once per week in order to evaluate the change in the infant's parent's mood resulting from following the bedtime regimen. Mood scores calculated for the Baseline and two Treatment Weeks and the scores are shown below in Table 14.

**Table 14. Results of Profile of Mood States**

| **Mood Scale** | **Baseline** | **Treatment Week 1** | **Treatment Week 2** |
|---|---|---|---|
| **Tension - Anxiety** | 7.52 | 5.54 | 3.38 |
| **Depression-Dejection** | 5.53 | 4.04 | 2.13 |
| **Anger-Hostility** | 3.9 | 3.07 | 1.2 |
| **Fatigue** | 9.22 | 6.75 | 3.78 |
| **Vigor** | 14.00 | 14.58 | 17.2 |
| **Confusion-Bewilderment** | 5.31 | 4.49 | 3.04 |

As shown in Tables 12, 13 and 14, the bedtime sensory regimen was effective in improving both the sleep behaviors of the infants as well as the mood state of their parents. In particular, as indicated by the significant improvements in the individual parameters, the regimens were extremely effective in improving many sleep behaviors including degree of pre-sleep fussiness, amount of time before falling asleep, number of night awakenings, amount of time during the night spent awake, length of continuous period of sleep, amount of time during the night spent sleeping, amount of time during the day spent sleeping, perception of sleep quality, mood state following sleep. Furthermore, the bedtime sensory regimens was effective in improving multiple mood states for the parents including the increase in vigor and the reduction of tension-anxiety, depression-dejection, anger-hostility, fatigue, and confusion-bewilderment.

While particular embodiments suitable for use in the method of the present invention have been described, it will be obvious to those skilled in the art that various changes and modifications of the present invention can be made without departing from the spirit and scope of the invention. It is intended to cover in the appended claims, all such modifications that are within the scope of this invention.

## Claims

1. A method for improving sleep behaviors of a human said method comprising the administration of a sensory regimen wherein said regimen comprises
a. the administration of a first sensory experience comprising topically applying to the skin or hair of said human a cleansing product containing an effective amount of a sensory fragrance; and
b. after step (a) the administration of a second sensory experience comprising administering an effective amount of a sensory fragrance,
wherein said sensory fragrance used in step (a) and said sensory fragrance used in step (b) is the same or different and wherein said sensory fragrance used in step (a) and said sensory fragrance used in step (b) is (I) capable of reducing alpha activity and (II) comprises:
i) from about 25% in total of at least five Relaxing Ingredients(R) selected from anethole, Bangalol.TM., basil oil, cis-hex-3-enol, coumarin, ethylene brassylate, ethyl linalol. Florosa.TM., Galaxolide.TM.. geraniol, cyclohexadecanolide, cyclopentadecanone, methyl anthranilate, alpha-iso-methyl ionone, Prunelia.TM., Silvanone.TM., alpha-terpineol, Traseolide.TM., Ultravanil.TM., gamma-undecalactone, vetiver oil, vetiver acetate, or mixtures thereof;
ii) from 0 to 45% in total of Non-Relaxing Ingredients (NR) selected from methyl nonyl aldehyde (aldehyde MNA), allyl amyl glycolate, acetyl cedrene, Amberlyn Super.TM., amyl salicylate, armoise oil, benzyl salicylate, bergamot oil, Bourgeonal.TM., cedar leaf oil, citronellol, beta-damascone, dimethyl benzyl carbinyl acetate. Ethyl Satranate.TM.. Everniate.TM., geranyl nitrile, Helional.TM., heliotropin, hexyl salicylate, lemon oil, Ligustral.TM., Lilial.TM., Lyral.TM., Mefrosol.TM., orange oil, orange terpenes, tagetes oil, tetrahydrogeraniol, vanillin, or mixtures thereof provided that the ratio of R to NR is at least 0.75;
iii) from 0 to 75% in total of Neutral Ingredients (N) selected from benzyl acetate, cassis base, Cyclamen Aldehyde.TM., carvone, cinnarnic alcohol, dihydroeugenol, dihydromyrcenol, eugenol, Extralide.TM., galbanum, gamma-decalactone, hydroxycitronellal, indole, isoeugenol, jasmin oil, Jasmopyrane Forte.TM., linalol, linalyl acetate, methyl dihydrojasmonate (MDJ), octahydrocoumarin, patchouli oil, 2-phenylethyl alcohol, rose oxide, rose oil, Sandalone.TM., Sandalore.TM., styrallyl acetate, ylang-ylang, or mixtures thereof;
iv) from 0 to 25% of at least one other perfumery material (M) selected from perfumery materials different from said ingredients (a) through (c) above, excluding odourless or low-odour solvents or diluents used as vehicles for other perfume components provided that the weight ratio R to (M+NR) exceeds 0.75;
wherein all percentages are based on weight of the sensory fragrance.

2. A method according to claim 1, wherein said first sensory experience is administered for at least five minutes.

3. A method according to claim 2, wherein said first sensory experience is administered for at least ten minutes.

4. A method according to claim 1, wherein said second sensory experience is administered for at least one minute.

5. A method according to claim 4, wherein said second sensory experience is administered for at least two minutes.

6. A method according to claim 5, wherein said second sensory experience is administered for at least five minutes.

7. A method according to claim 1, wherein said sleep behaviors are selected from at least one of (a) perception of sleep quality; (b) a composite sleep quality score (c) perceived sleep depth; (d) number of night awakenings; (e) latency to sleep; and (f) combinations thereof.

8. A method according to claim 7, wherein said sleep behaviors are assessed through (1) a questionnaire; (2) an interview; (3) a psychometric assessment tool; (4) a biometric assessment tool; or (5) a combination thereof.

9. A method according to claim 1. wherein said human is of an age ranging from two months to eighteen years old.

10. A method according to claim 9, wherein said human is of an age ranging from two months to twelve years old.

11. A method according to claim 10, wherein said human is of an age ranging from two months to six years old.

12. A method according to claim 11. wherein said human is of an age ranging from two months to three years.

13. A method according to claim 12, wherein said human is of an age ranging from about two months to eighteen months.

14. A method according to claim 11, wherein said human is of an age ranging from two months to twelve months.

15. A method according to claim 12 wherein said sleep behaviors are selected from at least one of the following:
length of continuous period of sleep
degree of pre-sleep fussiness
ratio of amount of night time to amount of day time sleep
number of night awakenings
amount of time before falling asleep
amount of time during the night spent awake
amount of time during the night spent sleeping
amount of time during the day spent sleeping
mood of the human following sleep
perception of sleep quality and
composite sleep quality.

16. A method according to claim 15, wherein said human is of an age ranging from seven months to fifteen months.

17. A method according to claim 15, wherein said perception of sleep quality is the result of a parent's assessment of said human's sleep behavior.

18. A method according to claim 17, wherein said parent's assessment of said human's sleep behavior is the result of (1) a questionnaire completed by said parent; (2) an interview with said parent; (3) a psychometric assessment tool; (4) a biometric assessment tool; or (5) a combination thereof.

19. A method according to claim 1, wherein the sensory fragrance used in said second sensory experience is the same sensory fragrance used in the first sensory experience.

20. The method according to claim 1, wherein said sensory fragrance is present in the cleansing product in an amount ranging from about 0.1 to about 1.5 percent by weight, based on the total weight of the cleansing product.

21. The method according to claim 20, wherein said sensory fragrance is present in the cleansing product in an amount ranging from about 0.4 to about 1.0 percent by weight, based on the total weight of the cleansing product.

22. The method according to claim 21, wherein said sensory fragrance is present in the cleansing product in an amount ranging from about 0.4 to about 0.6 percent by weight, based on the total weight of the cleansing product.

23. The method according to claim 1. wherein said secondary sensory experience comprises the administration of a leave-on personal care composition containing an effective amount of said sensory fragrance.

24. The method according to claim 23, wherein said sensory fragrance is the same sensory fragrance present in the cleansing product.

25. The method according to claim 23, wherein said leave-on personal care composition is a skin moisturizing composition.

26. The method according to claim 25, wherein said leave-on composition comprises from about 0.1 to about 1.0 by weight, based on the total weight of said leave-on composition, of said sensory fragrance.

27. The method according to claim 26, wherein said leave-on composition comprises from about 0.4 to about 0.6 by weight, based on the total weight of said leave-on composition, of said sensory fragrance.

28. The method according to claim 23, wherein said leave-on personal care composition is massaged into the skin or hair of said human.

29. A method for improving the mood state of a parent of a human child of an age ranging from two months to six years old, said method comprising the administration of a sensory regimen to said child wherein said regimen comprises:
(a) the administration of a first sensory experience comprising topically applying to the skin of said child a cleansing product containing an effective amount of a sensory fragrance; and
(b) after step (a) the administration of a second sensory experience comprising administering an effective amount of a sensory fragrance,
wherein said sensory fragrance used in step (a) and said sensory fragrance used in step (b) is the same or different and wherein said sensory fragrance used in step (a) and step (b) is (I) capable of reducing alpha activity and (II) comprises:
a) from about 25% in total of at least five Relaxing Ingredients(R) selected from anethole, Bangalol.TM., basil oil, cis-hex-3-enol, coumarin, ethylene brassylate, ethyl linalol, Florosa.TM., Galaxolide.TM., geraniol, cyclohexadecanolide, cyclopentadecanone, methyl anthranilate, alpha-iso-methyl ionone, Prunella.TM., Silvanone.TM., alpha-terpineol, Traseolide.TM., Ultravanil.TM., gamma-undecalactone, vetiver oil, vetiver acetate, or mixtures thereof;
b) from 0 to 45% in total of Non-Relaxing Ingredients (NR) selected from methyl nonyl aldehyde (aldehyde MNA), allyl amyl glycolate, acetyl cedrene, Amberlyn Super.TM., amyl salicylate, armoise oil, benzyl salicylate, bergamot oil, Bourgeonal.TM., cedar leaf oil, citronellol, beta-damascone, dimethyl benzyl carbinyl acetate, Ethyl Safranate.TM., Everniate.TM., geranyl nitrile, Helional.TM., heliotropin, hexyl salicylate, lemon oil, Ligustral.TM., Lilial.TM., Lyral.TM., Mefrosol.TM., orange oil, orange terpenes, tagetes oil, tetrahydrogeraniol, vanillin, or mixtures thereof provided that the ratio of R to NR is at least 0.75;
c) from 0 to 75% in total of Neutral Ingredients (N) selected from benzyl acetate, cassis base, Cyclamen Aldehyde.TM., carvone, cinnarnic alcohol, dihydroeugenol, dihydromyrcenol, eugenol, Extralide.TM., galbanum, gamma-decalactone, hydroxycitronellal, indole, isoeugenol, jasmin oil, Jasmopyrane Forte.TM., linalol, linalyl acetate, methyl dihydrojasmonate (MDJ), octahydrocoumarin, patchouli oil, 2-phenylethyl alcohol, rose oxide, rose oil, Sandalone.TM., Sandalore.TM., styrallyl acetate, ylang-ylang, or mixtures thereof;
d) from 0 to 25% of at least one other perfumery material (M) selected from perfumery materials different from said ingredients (a) through (c) above, excluding odourless or low-odour solvents or diluents used as vehicles for other perfume components provided that the weight ratio R to (M+NR) exceeds 0.75;
wherein all percentages are based on weight of the sensory fragrance; and
wherein said improvement in mood state is selected from at least one of the following:
(i) reduced tension-anxiety;
(ii) reduced anger-hostility;
(iii) reduced fatigue-inertia;
(iv) reduced confusion-bewilderment;
(v) reduced depression-dejection;
(vi) increased vigor-activity and
(vii) combinations thereof.

30. The method according to claim 20, wherein said human child is a child ranging between the ages of two to eighteen months.

31. A method for improving sleep behaviors of a human said method comprising the administration of a sensory regimen wherein said regimen comprises
a. the administration of a first sensory experience comprising topically applying to the skin or hair of said human a cleansing product containing an effective amount of a sensory fragrance; and
b. after step (a) the administration of a second sensory experience comprising administering an effective amount of a sensory fragrance ,
wherein said sensory fragrance used in step (a) and said sensory fragrance used in step (b) is the same or different and wherein said sensory fragrance used in step (a) and step (b) is capable of reducing alpha activity.

32. A kit comprising (a) a cleansing product containing an effective amount of a sensory fragrance and (b) a personal care product other than a cleansing product containing an effective amount of a sensory fragrance, wherein said sensory fragrance used in (a) and said sensory fragrance used in (b) is the same or different and wherein said sensory fragrance used in step (a) and step (b) is (I) capable of reducing alpha activity and (II) comprises:
i) from about 25% in total of at least five Relaxing Ingredients(R) selected from anethole, Bangalol.TM., basil oil, cis-hex-3-enol, coumarin, ethylene brassylate, ethyl linalol, Florosa.TM., Galaxolide.TM., geraniol, cyclohexadecanolide, cyclopentadecanone, methyl anthranilate, alpha-iso-methyl ionone, Prunella.TM., Silvanone.TM., alpha-terpineol, Traseolide.TM., Ultravanil.TM., gamma-undecalactone, vetiver oil, vetiver acetate, or mixtures thereof;
ii) from 0 to 45% in total of Non-Relaxing Ingredients (NR) selected from methyl nonyl aldehyde (aldehyde MNA), allyl amyl glycolate, acetyl cedrene, Amberlyn Super.TM., amyl salicylate, armoise oil, benzyl salicylate, bergamot oil, Bourgeonal.TM., cedar leaf oil, citronellol, beta-damascone, dimethyl benzyl carbinyl acetate, Ethyl Safranate.TM., Everniate.TM., geranyl nitrile, Helional.TM., heliotropin, hexyl salicylate, lemon oil, Ligustral.TM., Lilial.TM., Lyral.TM., Mefrosol.TM., orange oil, orange terpenes, tagetes oil, tetrahydrogeraniol, vanillin, or mixtures thereof provided that the ratio of R to NR is at least 0.75;
iii) from 0 to 75% in total of Neutral Ingredients (N) selected from benzyl acetate, cassis base, Cyclamen Aldehyde.TM., carvone, cinnarnic alcohol, dihydroeugenol, dihydromyrcenol, eugenol, Extralide.TM., galbanum, gamma-decalactone, hydroxycitronellal, indole, isoeugenol, jasmin oil, Jasmopyrane Forte.TM., linalol, linalyl acetate, methyl dihydrojasmonate (MDJ), octahydrocoumarin, patchouli oil, 2-phenylethyl alcohol, rose oxide, rose oil, Sandalone.TM., Sandalore.TM., styrallyl acetate, ylang-ylang, or mixtures thereof;
iv) from 0 to 25% of at least one other perfumery material (M) selected from perfumery materials different from said ingredients (a) through (c) above, excluding odourless or low-odour solvents or diluents used as vehicles for other perfume components provided that the weight ratio R to (M+NR) exceeds 0.75;
wherein all percentages are based on weight of the sensory fragrance.
